# EUROPEAN PATENT APPLICATION

(11) **EP 1 375 624 A1**
(43) Date of publication of application: **02.01.2004**
(21) Application number: 03076861.8
(22) Date of filing: 16.06.2003
(51) Int. Cl.: C09K 11/06, H05B 33/14, H01L 51/20

(54) **Device containing green organic light-emitting diode**

(30) Priority: 27.06.2002 US 184356; 23.09.2002 US 252487
(71) Applicant: EASTMAN KODAK COMPANY, Rochester, New York 14650 (US)
(72) Inventor: Cosimbescu, Lelia, c/o Eastman Kodak Company, Rochester, New York 14650-2201 (US)
(74) Representative: Haile, Helen Cynthia

(57) **Abstract**

Disclosed is an OLED device comprising a non-gallium host compound and a green light emitting dopant wherein the dopant comprises an N,N'-diarylquinacridone compound optionally containing on the two aryl groups and the quinacridone nucleus only substituent groups having Hammett's σ constant values at least 0.05 more positive than that for a corresponding methyl group, such substituent groups including up to two substituent groups directly on the carbon members of the quinacridone nucleus, provided that said substituent groups do not form a ring fused to the five-ring quinacridone nucleus. Such a device exhibits improved stability, and at the same time, provides high efficiency and good color.

## Description

This invention relates to organic electroluminescent (EL) devices. More specifically, this invention relates to EL devices containing an organic green light emitting diode dopant that comprises a certain N,N'-diarylquinacridone compound exhibiting high efficiency, good color and high stability.

While organic electroluminescent (EL) devices have been known for over two decades, their performance limitations have represented a barrier to many desirable applications. In simplest form, an organic EL device is comprised of an anode for hole injection, a cathode for electron injection, and an organic medium sandwiched between these electrodes to support charge recombination that yields emission of light. These devices are also commonly referred to as organic light-emitting diodes, or OLEDs. Representative of earlier organic EL devices are Gurnee et al. U.S. Pat. No. 3,172,862, issued Mar. 9, 1965; Gurnee U.S. Pat. No. 3,173,050, issued Mar. 9, 1965; Dresner, "Double Injection Electroluminescence in Anthracene", RCA Review, Vol. 30, pp. 322-334,1969; and Dresner U.S. Pat. No. 3,710,167, issued Jan. 9, 1973. The organic layers in these devices, usually composed of a polycyclic aromatic hydrocarbon, were very thick (much greater than 1 µm). Consequently, operating voltages were very high, often >100V.

More recent organic EL devices include an organic EL element consisting of extremely thin layers (e.g. <1.0 µm) between the anode and the cathode. Herein, the term "organic EL element" encompasses the layers between the anode and cathode electrodes. Reducing the thickness lowered the resistance of the organic layer and has enabled devices that operate much lower voltage. In a basic two-layer EL device structure, described first in US 4,356,429, one organic layer of the EL element adjacent to the anode is specifically chosen to transport holes, therefore, it is referred to as the hole-transporting layer, and the other organic layer is specifically chosen to transport electrons, referred to as the electron-transporting layer. Recombination of the injected holes and electrons within the organic EL element results in efficient electroluminescence.

There have also been proposed three-layer organic EL devices that contain an organic light-emitting layer (LEL) between the hole- transporting layer and electron-transporting layer, such as that disclosed by Tang et al [*J. Applied Physics,* Vol. 65, Pages 3610-3616, 1989]. The light-emitting layer commonly consists of a host material doped with a guest material. Still further, there has been proposed in US 4,769,292 a four-layer EL element comprising a hole-injecting layer (HIL), a hole-transporting layer (HTL), a light-emitting layer (LEL) and an electron transport/injection layer (ETL). These structures have resulted in improved device efficiency.

It has been found that certain gallium compounds present undesirable risks including, for example, high toxicity of gallium arsenide. Such compounds are thus generally objectionable as hosts in OLED devices.

Quinacridones have been studied as emissive dopants for OLED devices, e.g., as described in US 5,227,252, JP 09-13026, US 5,593,788, JP 11-54283, and JP 11-67449. US 5,593,788 teaches that substitution on the nitrogen of the quinacridone improves stability.

However, the stability of quinacridone derivatives as taught in the prior art is not sufficient for various applications. Thus, there is still a need for green-emitting devices with higher stability, and at the same time, providing high efficiency and good color.

The invention provides an OLED device comprising a non-gallium host compound and a green light emitting dopant wherein the dopant comprises an N,N'-diarylquinacridone compound optionally containing on the two aryl groups and the quinacridone nucleus only substituent groups having Hammett's σ constant values at least 0.05 more positive than that for a corresponding methyl group, such substituent groups including up to two substituent groups directly on the carbon members of the quinacridone nucleus, provided that said substituent groups do not form a ring fused to the five-ring quinacridone nucleus.

The device of the invention exhibits improved stability, and at the same time, provides high efficiency and good color. An advantage of this invention is that green OLEDs can be used in a wider variety of applications that require high efficiency and high stability. This results in greatly increasing overall lifetime of the display device it is used in. It is another advantage that the emissive material is easy to synthesize and purify.

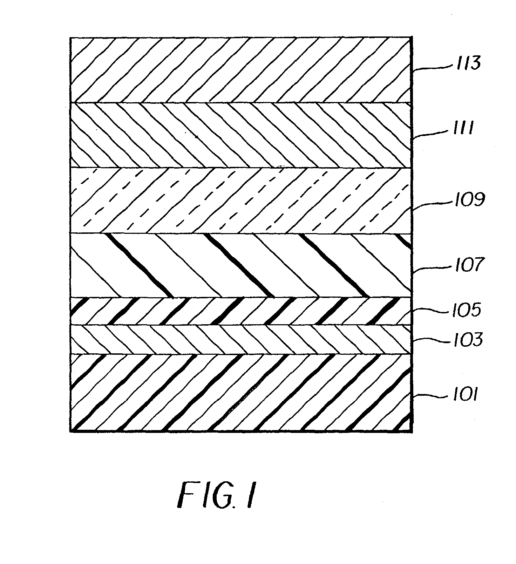

FIG. 1 shows a schematic cross-section of an OLED device of this invention.

The invention is summarized above. The device comprises a non-gallium host compound. The host suitably comprises, for example, an aluminum complex, an anthracene compound, or a distyrylarylene derivative. These materials are exemplified by Aluminum trisoxine [alias, tris(8-quinolinolato)aluminum(III)] (Alq), include 9,10-di-(2-naphthyl)anthracene (ADN) and 2-*t*-butyl-9,10-di-(2-naphthyl)anthracene (TBADN), as more fully described hereafter.

The green light emitting dopant comprises an N,N'-diarylquinacridone compound optionally containing on the two aryl groups and the quinacridone nucleus only substituent groups having Hammett's σ constant values at least 0.05 more positive than that for a corresponding methyl group, such substituent groups including up to two substituent groups directly on the carbon members of the quinacridone nucleus, provided that said substituent groups do not form a ring fused to the five-ring quinacridone nucleus. The Hammett's constant measures the relative electron withdrawing ability of a substituent on an aryl ring with more positive values being more electron withdrawing. Values are given in numerous handbooks such as Substituent Constants for Correlation Analysis in Chemistry and Biology, C. Hansch and A.J. Leo, Wiley, New York (1979) and pKa Prediction for Organic Acids and Bases D.D.Perrin, B. Dempsey, and E.P.Serjeant, Chapman and Hall, New York (1981). Most groups other than alkyl, alkoxy, hydroxy and amine groups satisfy this requirement and are thus permissible substituents. Unsubstituted N,N'-diarylquinacridone is a compound useful in the invention. Conveniently used are dopants where the diaryl groups are diphenyl groups.

When substituents are present that have a Hammett's σ constant value that is not at least 0.05 more positive than that for a corresponding methyl group, the combination results are unsatisfactory, as shown in Table 1. Thus, such substituents are not optionally permitted.

When substituent groups are employed, they may include up to two substituent groups on the carbon members of the quinacridone nucleus. Greater numbers do not provide further advantages, are more complicated to synthesize, and tend to adversely affect color.

The device of the invention preferably incorporates substituents that are selected so that the device emits green light having a CIEx value less than 0.35, a CIEy value greater than 0.62, and a luminance efficiency greater than 7 cd/A when applied with a current density of 20 mA/cm².

The dopant suitably has the following formula I: 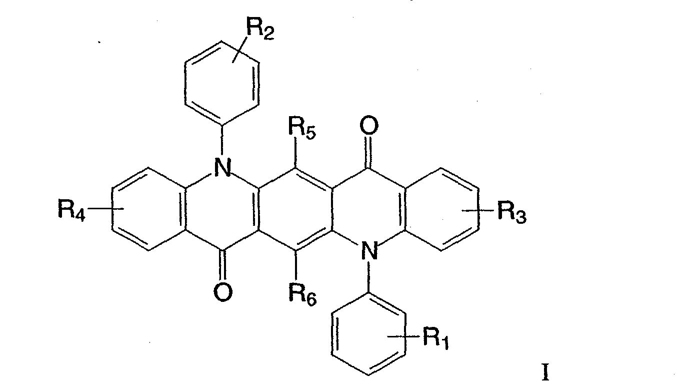 wherein, R₁ and R₂ represent one or more independently selected hydrogen or substituent groups having Hammett's σ constant values at least 0.05 more positive than that for a corresponding methyl group and each of R₃ through R₆ represents hydrogen or up to two substituents as selected for R₁ above. Suitably, R₁ and R₂ are hydrogen or independently selected from halogen, aryl, an aromatic heterocycle, or a fused aromatic or heteroaromatic ring and R₃ through R₆ represent hydrogen or one or more substituents independently selected from halogen, aryl, and an aromatic heterocycle. R₁ - R₆ are independently selected to include hydrogen, phenyl, biphenyl, or naphthyl groups.

It is desirable that the substituents on the quinacridone nucleus not form a ring fused to the five-ring quinacridone nucleus. Such rings typically adversely affect either stability or color depending on the aromatic or alicyclic nature of the fused ring.

If desired, the substituents may themselves be further substituted one or more times with the described substituent groups. The particular substituents used may be selected by those skilled in the art to attain the desired desirable properties for a specific application and can include, for example, electron-withdrawing groups and steric groups. Except as provided above, when a molecule may have two or more substituents, the substituents may be joined together to form a ring such as a fused ring unless otherwise provided. Generally, the above groups and substituents thereof may include those having up to 48 carbon atoms, typically 1 to 36 carbon atoms and usually less than 24 carbon atoms, but greater numbers are possible depending on the particular substituents selected.

Useful compounds in this invention include:

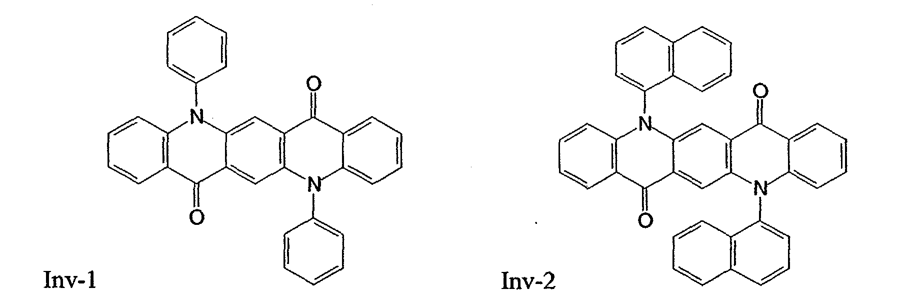

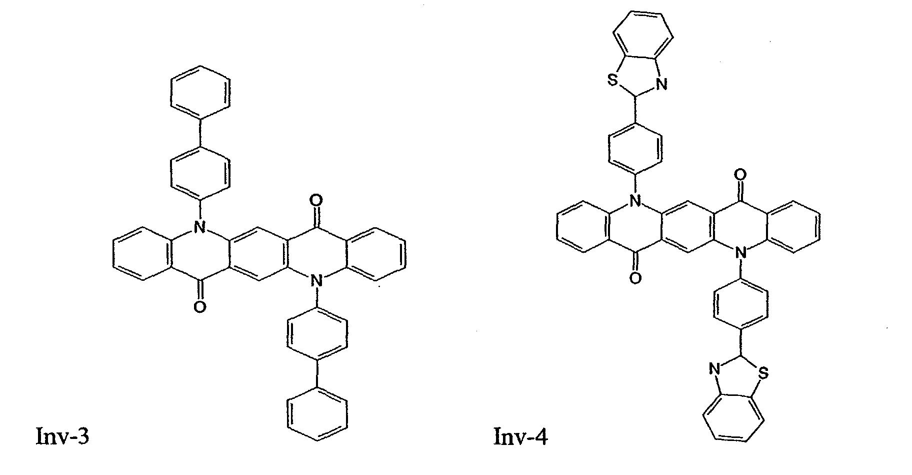

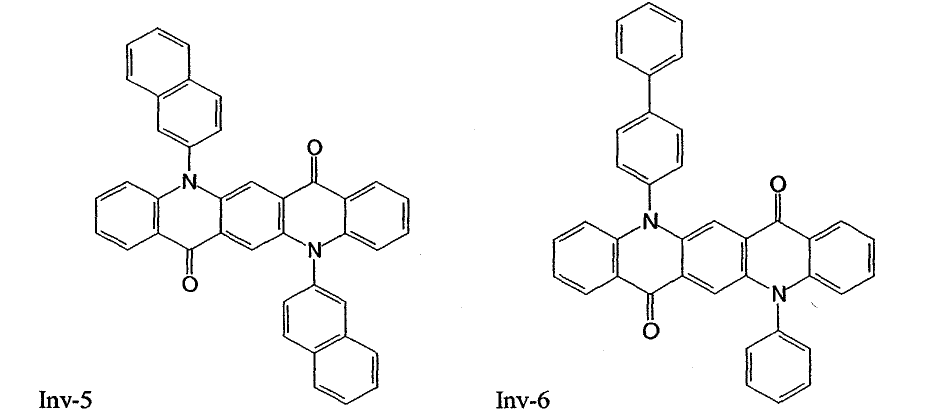

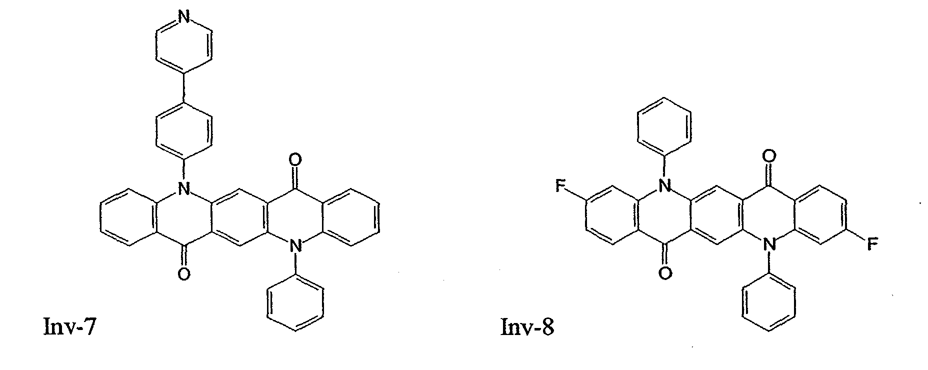

The host/dopants are typically employed in a light-emitting layer comprising some amount of the inventive compound molecularly dispersed in a host as defined below. Examples of useful host materials (defined below) include Alq, ADN, TBADN, distyrylarylene derivatives and mixtures thereof. Quinacridone derivatives of this invention are typically used, typically less than 10%, less than 5%, or less than 2% with amounts of 0.1 to 1% weight ratio to host usually employed.

### General device architecture

The present invention can be employed in most OLED device configurations. These include very simple structures comprising a single anode and cathode to more complex devices, such as passive matrix displays comprised of orthogonal arrays of anodes and cathodes to form pixels, and active-matrix displays where each pixel is controlled independently, for example, with thin film transistors (TFTs).

There are numerous configurations of the organic layers wherein the present invention can be successfully practiced. A typical structure is shown in Figure 1 and is comprised of a substrate **101**, an anode **103**, a hole-injecting layer **105**, a hole-transporting layer **107**, a light-emitting layer **109**, an electron-transporting layer **111**, and a cathode **113**. These layers are described in detail below. Note that the substrate may alternatively be located adjacent to the cathode, or the substrate may actually constitute the anode or cathode. The organic layers between the anode and cathode are conveniently referred to as the organic EL element. Also, the total combined thickness of the organic layers is preferably less than 500 nm.

The OLED is operated by applying a potential between the anode and cathode such that the anode is at a more positive potential than the cathode. Holes are injected into the organic EL element from the anode and electrons are injected into the organic EL element at the anode. Enhanced device stability can sometimes be achieved when the OLED is operated in an AC mode where, for some time period in the cycle, the potential bias is reversed and no current flows. An example of an AC driven OLED is described in US 5,552,678.

### Substrate

The OLED device of this invention is typically provided over a supporting substrate **101** where either the cathode or anode can be in contact with the substrate. The electrode in contact with the substrate is conveniently referred to as the bottom electrode. Conventionally, the bottom electrode is the anode, but this invention is not limited to that configuration. The substrate can either be light transmissive or opaque, depending on the intended direction of light emission. The light transmissive property is desirable for viewing the EL emission through the substrate. Transparent glass or plastic is commonly employed in such cases. The substrate may be a complex structure comprising multiple layers of materials. This is typically the case for active matrix substrates wherein TFTs are provided below the OLED layers. It is still necessary that the substrate, at least in the emissive pixilated areas, be comprised of largely transparent materials such as glass or polymers. For applications where the EL emission is viewed through the top electrode, the transmissive characteristic of the bottom support is immaterial, and therefore can be light transmissive, light absorbing or light reflective. Substrates for use in this case include, but are not limited to, glass, plastic, semiconductor materials, silicon, ceramics, and circuit board materials. Again, the substrate may be a complex structure comprising multiple layers of materials such as found in active matrix TFT designs. Of course it is necessary to provide in these device configurations a light-transparent top electrode.

### Anode

When EL emission is viewed through anode **103**, the anode should be transparent or substantially transparent to the emission of interest. Common transparent anode materials used in this invention are indium-tin oxide (ITO), indium-zinc oxide (IZO) and tin oxide, but other metal oxides can work including, but not limited to, aluminum- or indium-doped zinc oxide, magnesium-indium oxide, and nickel-tungsten oxide. In addition to these oxides, metal nitrides, such as gallium nitride, and metal selenides, such as zinc selenide, and metal sulfides, such as zinc sulfide, can be used as the anode **103**. For applications where EL emission is viewed only through the cathode electrode, the transmissive characteristics of anode are immaterial and any conductive material can be used, transparent, opaque or reflective. Example conductors for this application include, but are not limited to, gold, iridium, molybdenum, palladium, and platinum. Typical anode materials, transmissive or otherwise, have a work function of 4.1 eV or greater. Desired anode materials are commonly deposited by any suitable means such as evaporation, sputtering, chemical vapor deposition, or electrochemical means. Anodes can be patterned using well-known photolithographic processes. Optionally, anodes may be polished prior to application of other layers to reduce surface roughness so as to minimize shorts or enhance reflectivity.

### Hole-Injecting Layer (HIL)

While not always necessary, it is often useful that a hole-injecting layer **105** be provided between anode **103** and hole-transporting layer **107**. The hole-injecting material can serve to improve the film formation property of subsequent organic layers and to facilitate injection of holes into the hole-transporting layer. Suitable materials for use in the hole-injecting layer include, but are not limited to, porphyrinic compounds as described in US 4,720,432, plasma-deposited fluorocarbon polymers as described in US 6,208,075, and some aromatic amines, for example, m-MTDATA (4,4',4"-tris[(3-methylphenyl)phenylamino]triphenylamine). Alternative hole-injecting materials reportedly useful in organic EL devices are described in EP 0 891 121 A1 and EP 1 029 909 A1.

### Hole-Transporting Layer (HTL)

The hole-transporting layer **107** of the organic EL device contains at least one hole-transporting compound such as an aromatic tertiary amine, where the latter is understood to be a compound containing at least one trivalent nitrogen atom that is bonded only to carbon atoms, at least one of which is a member of an aromatic ring. In one form the aromatic tertiary amine can be an arylamine, such as a monoarylamine, diarylamine, triarylamine, or a polymeric arylamine. Exemplary monomeric triarylamines are illustrated by Klupfel et al. US 3,180,730. Other suitable triarylamines substituted with one or more vinyl radicals and/or comprising at least one active hydrogen containing group are disclosed by Brantley et al US 3,567,450 and US 3,658,520.

A more preferred class of aromatic tertiary amines are those which include at least two aromatic tertiary amine moieties as described in US 4,720,432 and US 5,061,569. Such compounds include those represented by structural formula (A). 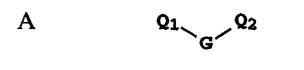 wherein Q₁ and Q₂ are independently selected aromatic tertiary amine moieties and G is a linking group such as an arylene, cycloalkylene, or alkylene group of a carbon to carbon bond. In one embodiment, at least one of Q₁ or Q₂ contains a polycyclic fused ring structure, e.g., a naphthalene. When G is an aryl group, it is conveniently a phenylene, biphenylene, or naphthalene moiety.

A useful class of triarylamines satisfying structural formula (A) and containing two triarylamine moieties is represented by structural formula (B): 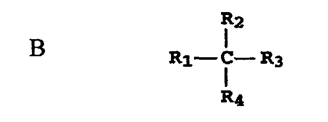 where
R₁ and R₂ each independently represents a hydrogen atom, an aryl group, or an alkyl group or R₁ and R₂ together represent the atoms completing a cycloalkyl group; and
R₃ and R₄ each independently represents an aryl group, which is in turn substituted with a diaryl substituted amino group, as indicated by structural formula (C): 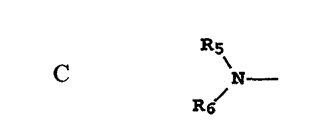 wherein R₅ and R₆ are independently selected aryl groups. In one embodiment, at least one of R₅ or R₆ contains a polycyclic fused ring structure, e.g., a naphthalene.

Another class of aromatic tertiary amines are the tetraaryldiamines. Desirable tetraaryldiamines include two diarylamino groups, such as indicated by formula (C), linked through an arylene group. Useful tetraaryldiamines include those represented by formula (D). 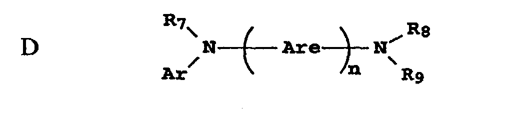 wherein
each Are is an independently selected arylene group, such as a phenylene or anthracene moiety,
n is an integer of from 1 to 4, and
Ar, R₇, R₈, and R₉ are independently selected aryl groups.
In a typical embodiment, at least one of Ar, R₇, R₈, and R₉ is a polycyclic fused ring structure, e.g., a naphthalene

The various alkyl, alkylene, aryl, and arylene moieties of the foregoing structural formulae (A), (B), (C), (D), can each in turn be substituted. Typical substituents include alkyl groups, alkoxy groups, aryl groups, aryloxy groups, and halogen such as fluoride, chloride, and bromide. The various alkyl and alkylene moieties typically contain from I to 6 carbon atoms. The cycloalkyl moieties can contain from 3 to 10 carbon atoms, but typically contain five, six, or seven ring carbon atoms--e.g., cyclopentyl, cyclohexyl, and cycloheptyl ring structures. The aryl and arylene moieties are usually phenyl and phenylene moieties.

The hole-transporting layer can be formed of a single or a mixture of aromatic tertiary amine compounds. Specifically, one may employ a triarylamine, such as a triarylamine satisfying the formula (B), in combination with a tetraaryldiamine, such as indicated by formula (D). When a triarylamine is employed in combination with a tetraaryldiamine, the latter is positioned as a layer interposed between the triarylamine and the electron injecting and transporting layer. Illustrative of useful aromatic tertiary amines are the following:
1,1 -Bis(4-di-*p*-tolylaminophenyl)cyclohexane
1,1 -Bis(4-di-*p*-tolylaminophenyl)-4-phenylcyclohexane
4,4'-Bis(diphenylamino)quadriphenyl
Bis(4-dimethylamino-2-methylphenyl)-phenylmethane
N,N,N-Tri(*p*-tolyl)amine
4-(di-p-tolylamino)-4'-[4(di-*p*-tolylamino)-styryl]stilbene
N,N,N',N'-Tetra-*p*-tolyl-4-4'-diaminobiphenyl
N,N,N',N'-Tetraphenyl-4,4'-diaminobiphenyl
N,N,N',N'-tetra-1-naphthyl-4,4'-diaminobiphenyl
N,N,N',N'-tetra-2-naphthyl-4,4'-diaminobiphenyl
N-Phenylcarbazole
4,4'-Bis[N-(1-naphthyl)-N-phenyiamino]biphenyl
4,4'-Bis[N-(1-naphthyl)-N-(2-naphthyl)amino]biphenyl
4,4"-Bis[N-( 1-naphthyl)-N-phenylamino]p-terphenyl
4,4'-Bis[N-(2-naphthyl)-N-phenylamino]biphenyl
4,4'-Bis[N-(3-acenaphthenyl)-N-phenylamino]biphenyl
1,5-Bis[N-(1-naphthyl)-N-phenylamino]naphthalene
4,4'-Bis[N-(9-anthryl)-N-phenylamino]biphenyl
4,4"-Bis[N-(1-anthryl)-N-phenylamino]-*p*-terphenyl
4,4'-Bis[N-(2-phenanthryl)-N-phenylamino]biphenyl
4,4'-Bis[N-(8-fluoranthenyl)-N-phenylamino]biphenyl
4,4'-Bis[N-(2-pyrenyl)-N-phenylamino]biphenyl
4,4'-Bis[N-(2-naphthacenyl)-N-phenylamino]biphenyl
4,4'-Bis[N-(2-perylenyl)-N-phenylamino]biphenyl
4,4'-Bis[N-(1-coronenyl)-N-phenylamino]biphenyl
2,6-Bis(di-*p*-tolylamino)naphthalene
2,6-Bis[di-(1-naphthyl)amino]naphthalene
2,6-Bis[N-(1-naphthyl)-N-(2-naphthyl)amino]naphthalene
N,N,N',N'-Tetra(2-naphthyl)-4,4"-diamino-*p*-terphenyl
4,4'-Bis{N-phenyl-N-[4-(1-naphthyl)-phenyl]amino}biphenyl
4,4'-Bis[N-phenyl-N-(2-pyrenyl)amino]biphenyl
2,6-Bis[N,N-di(2-naphthyl)amine]fluorene
1,5-Bis[N-(1-naphthyl)-N-phenylamino]naphthalene
4,4',4"-tris[(3-methylphenyl)phenylamino]triphenylamine

Another class of useful hole-transporting materials includes polycyclic aromatic compounds as described in EP 1 009 041. Tertiary aromatic amines with more than two amine groups may be used including oligomeric materials. In addition, polymeric hole-transporting materials can be used such as poly(N-vinylcarbazole) (PVK), polythiophenes, polypyrrole, polyaniline, and copolymers such as poly(3,4-ethylenedioxythiophene) / poly(4-styrenesulfonate) also called PEDOT/PSS.

### Light-Emitting Layer (LEL)

This invention is primarily directed to the light-emitting layer (LEL). As described above, the compound of Formula 1 is commonly used along with a host to yield green emission. The green OLED of this invention may be used along with other dopants or LELs to alter the emissive color, e.g., to make white. In addition, the green OLED of this invention can be used along with other OLED devices to make full color display devices. Various aspects of the host of this invention and other OLED devices and dopants with which the inventive OLED can be used are described below.

As more fully described in U.S. Patent Nos. 4,769,292 and 5,935,721, the light-emitting layer (LEL) **109** of the organic EL element includes a luminescent or fluorescent material where electroluminescence is produced as a result of electron-hole pair recombination in this region. The light-emitting layer can be comprised of a single material, but more commonly consists of a host material doped with a guest compound or compounds where light emission comes primarily from the dopant and can be of any color. The host materials in the light-emitting layer can be an electron-transporting material, as defined below, a hole-transporting material, as defined above, or another material or combination of materials that support hole-electron recombination. The dopant is usually chosen from highly fluorescent dyes, but phosphorescent compounds, e.g., transition metal complexes as described in WO 98/55561, WO 00/18851, WO 00/57676, and WO 00/70655 are also useful. Dopants are typically coated as 0.01 to 10 % by weight into the host material. Polymeric materials such as polyfluorenes and polyvinylarylenes (e.g., poly(p-phenylenevinylene), PPV) can also be used as the host material. In this case, small molecule dopants can be molecularly dispersed into the polymeric host, or the dopant could be added by copolymerizing a minor constituent into the host polymer.

An important relationship for choosing a dye as a dopant is a comparison of the bandgap potential which is defined as the energy difference between the highest occupied molecular orbital and the lowest unoccupied molecular orbital of the molecule. For efficient energy transfer from the host to the dopant molecule, a necessary condition is that the band gap of the dopant is smaller than that of the host material. For phosphorescent emitters it is also important that the host triplet energy level of the host be high enough to enable energy transfer from host to dopant.

Host and emitting molecules known to be of use include, but are not limited to, those disclosed in US 4,768,292, US 5,141,671, US 5,150,006, US 5,151,629, US 5,405,709, US 5,484,922, US 5,593,788, US 5,645,948, US 5,683,823, US 5,755,999, US 5,928,802, US 5,935,720, US 5,935,721, and US 6,020,078.

Metal complexes of 8-hydroxyquinoline and similar derivatives (Formula E) constitute one class of useful host compounds capable of supporting electroluminescence, and are particularly suitable for light emission of wavelengths longer than 500 nm, e.g., green, yellow, orange, and red. 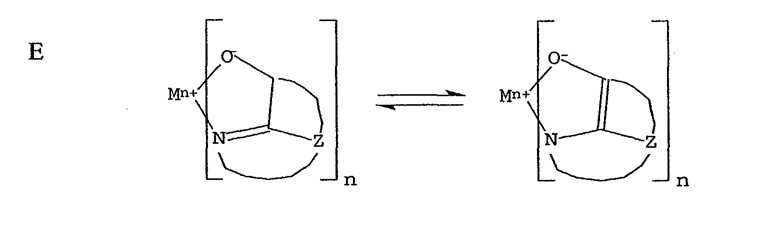 wherein
M represents a metal;
n is an integer of from 1 to 4; and
Z independently in each occurrence represents the atoms completing a nucleus having at least two fused aromatic rings.

From the foregoing it is apparent that the metal can be monovalent, divalent, trivalent, or tetravalent metal. The metal can, for example, be an alkali metal, such as lithium, sodium, or potassium; an alkaline earth metal, such as magnesium or calcium; an earth metal, such aluminum, or a transition metal such as zinc or zirconium. Generally any monovalent, divalent, trivalent, or tetravalent metal known to be a useful chelating metal can be employed.

Z completes a heterocyclic nucleus containing at least two fused aromatic rings, at least one of which is an azole or azine ring. Additional rings, including both aliphatic and aromatic rings, can be fused with the two required rings, if required. To avoid adding molecular bulk without improving on function the number of ring atoms is usually maintained at 18 or less.

Illustrative of useful chelated oxinoid compounds are the following:
CO-1: Aluminum trisoxine [alias, tris(8-quinolinolato)aluminum(III)] (Alq)
CO-2: Magnesium bisoxine [alias, bis(8-quinolinolato)magnesium(II)]
CO-3: Bis[benzo {f}-8-quinolinolato]zinc (II)
CO-4: Bis(2-methyl-8-quinolinolato)aluminum(III)-µ-oxo-bis(2-methyl-8-quinolinolato) aluminum(III)
CO-5: Indium trisoxine [alias, tris(8-quinolinolato)indium]
CO-6: Aluminum tris(5-methyloxine) [alias, tris(5-methyl-8-quinolinolato) aluminum(III)]
CO-7: Lithium oxine [alias, (8-quinolinolato)lithium(I)]
CO-8: Zirconium oxine [alias, tetra(8-quinolinolato)zirconium(IV)]

Derivatives of 9,10-di-(2-naphthyl)anthracene (Formula F) constitute one class of useful hosts capable of supporting electroluminescence, and are particularly suitable for light emission of wavelengths longer than 400 nm, e.g., blue, green, yellow, orange or red.

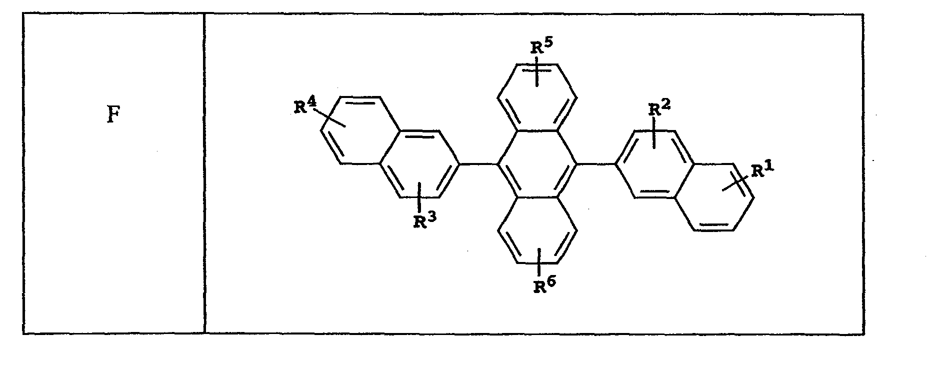

wherein: R¹, R², R³, R⁴, R⁵, and R⁶ represent one or more substituents on each ring where each substituent is individually selected from the following groups:
Group 1: hydrogen, or alkyl of from 1 to 24 carbon atoms;
Group 2: aryl or substituted aryl of from 5 to 20 carbon atoms;
Group 3: carbon atoms from 4 to 24 necessary to complete a fused aromatic ring of anthracenyl; pyrenyl, or perylenyl;
Group 4: heteroaryl or substituted heteroaryl of from 5 to 24 carbon atoms as necessary to complete a fused heteroaromatic ring of furyl, thienyl, pyridyl, quinolinyl or other heterocyclic systems;
Group 5: alkoxylamino, alkylamino, or arylamino of from 1 to 24 carbon atoms; and
Group 6: fluorine, chlorine, bromine or cyano.

Illustrative examples include 9,10-di-(2-naphthyl)anthracene (ADN) and 2-*t*-butyl-9,10-di-(2-naphthyl)anthracene (TBADN). Other anthracene derivatives can be useful as a host in the LEL, including derivatives of 9,10-bis[4-(2,2-diphenylethenyl)phenyl]anthracene. Mixtures of hosts can also be adventitious, such as mixtures of compounds of Formula E and Formula F.

Benzazole derivatives (Formula G) constitute another class of useful hosts capable of supporting electroluminescence, and are particularly suitable for light emission of wavelengths longer than 400 nm, e.g., blue, green, yellow, orange or red. 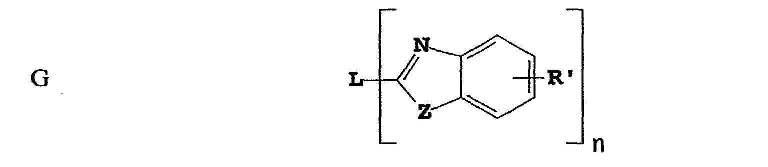 Where:
n is an integer of 3 to 8;
Z is O, NR or S; and
R and R' are individually hydrogen; alkyl of from 1 to 24 carbon atoms, for example, propyl, t-butyl, heptyl, and the like; aryl or hetero-atom substituted aryl of from 5 to 20 carbon atoms for example phenyl and naphthyl, furyl, thienyl, pyridyl, quinolinyl and other heterocyclic systems; or halo such as chloro, fluoro; or atoms necessary to complete a fused aromatic ring;
L is a linkage unit consisting of alkyl, aryl, substituted alkyl, or substituted aryl, which conjugately or unconjugately connects the multiple benzazoles together. An example of a useful benzazole is 2, 2', 2"-(1,3,5-phenylene)tris[1-phenyl-1H-benzimidazole].

Distyrylarylene derivatives are also useful hosts, as described in US 5,121,029. Carbazole derivatives are particularly useful hosts for phosphorescent emitters.

Useful fluorescent dopants include, but are not limited to, derivatives of anthracene, tetracene, xanthene, perylene, rubrene, coumarin, rhodamine, and quinacridone, dicyanomethylenepyran compounds, thiopyran compounds, polymethine compounds, pyrilium and thiapyrilium compounds, fluorene derivatives, periflanthene derivatives, indenoperylene derivatives, bis(azinyl)amine boron compounds, bis(azinyl)methane compounds, and carbostyryl compounds. Illustrative examples of useful dopants include, but are not limited to, the following:

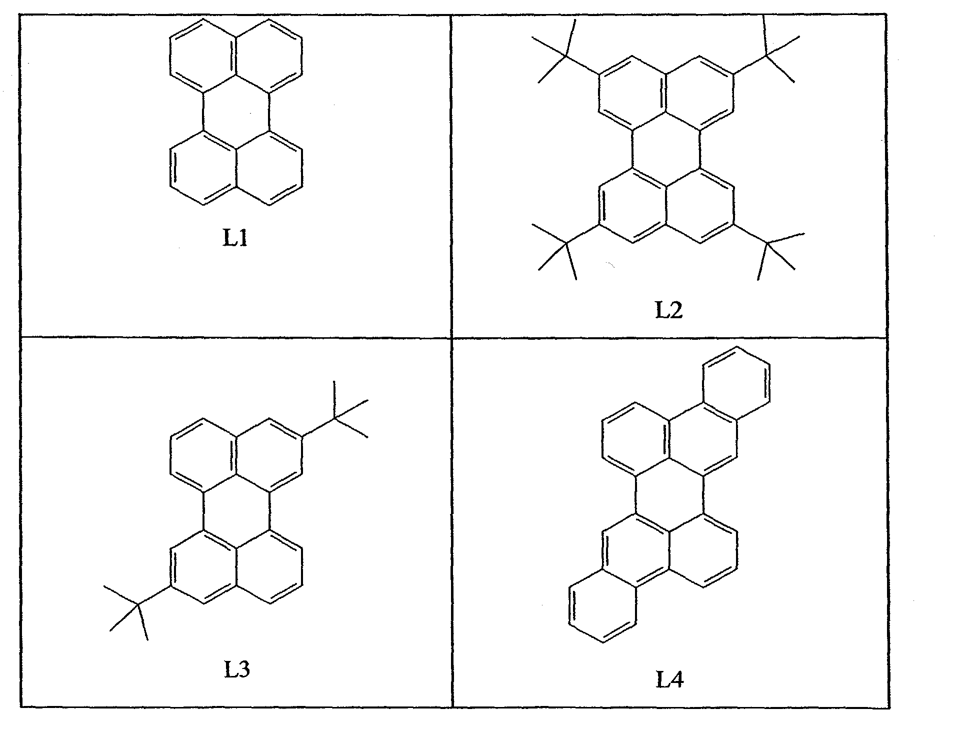

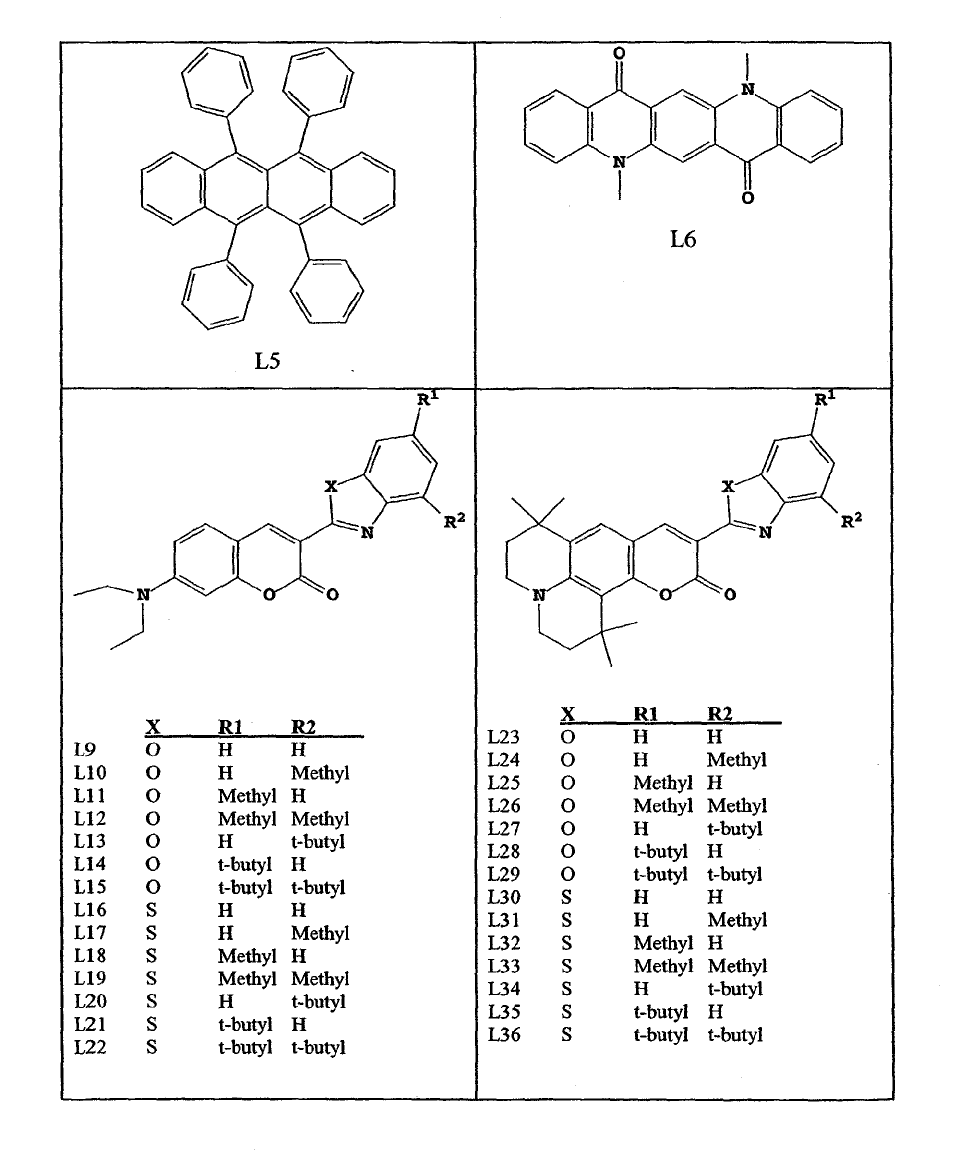

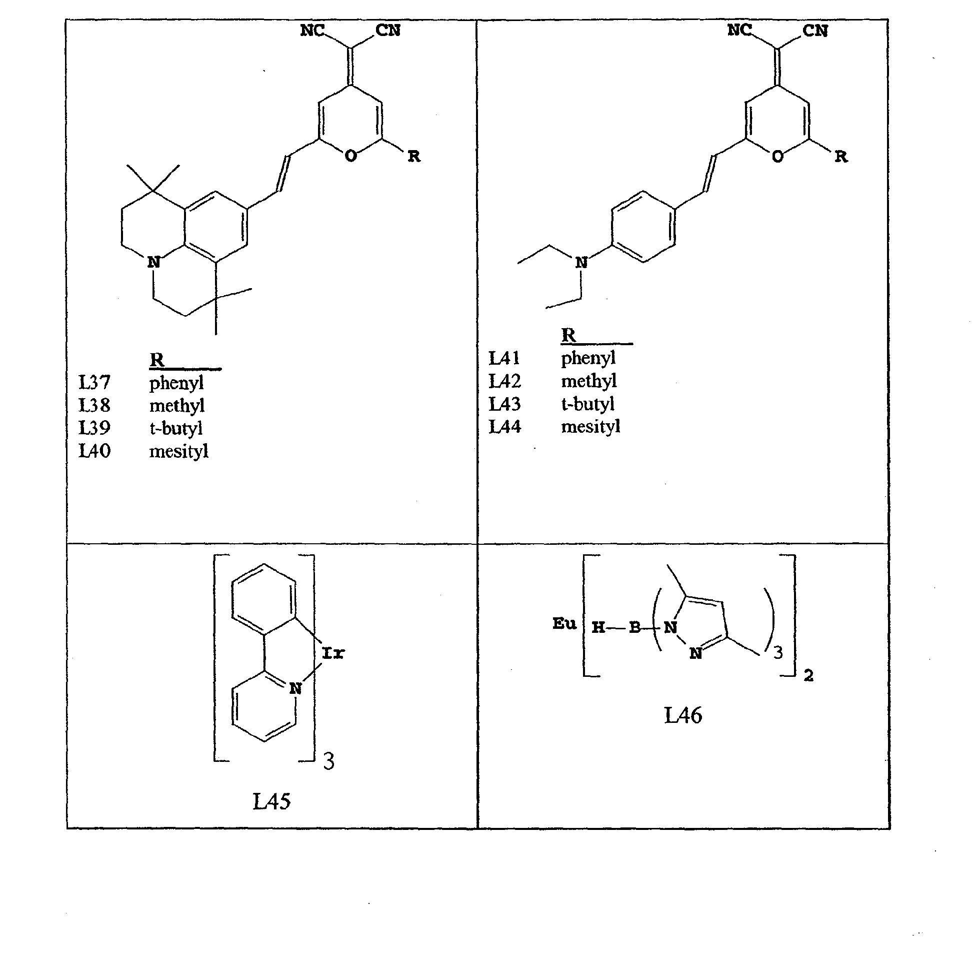

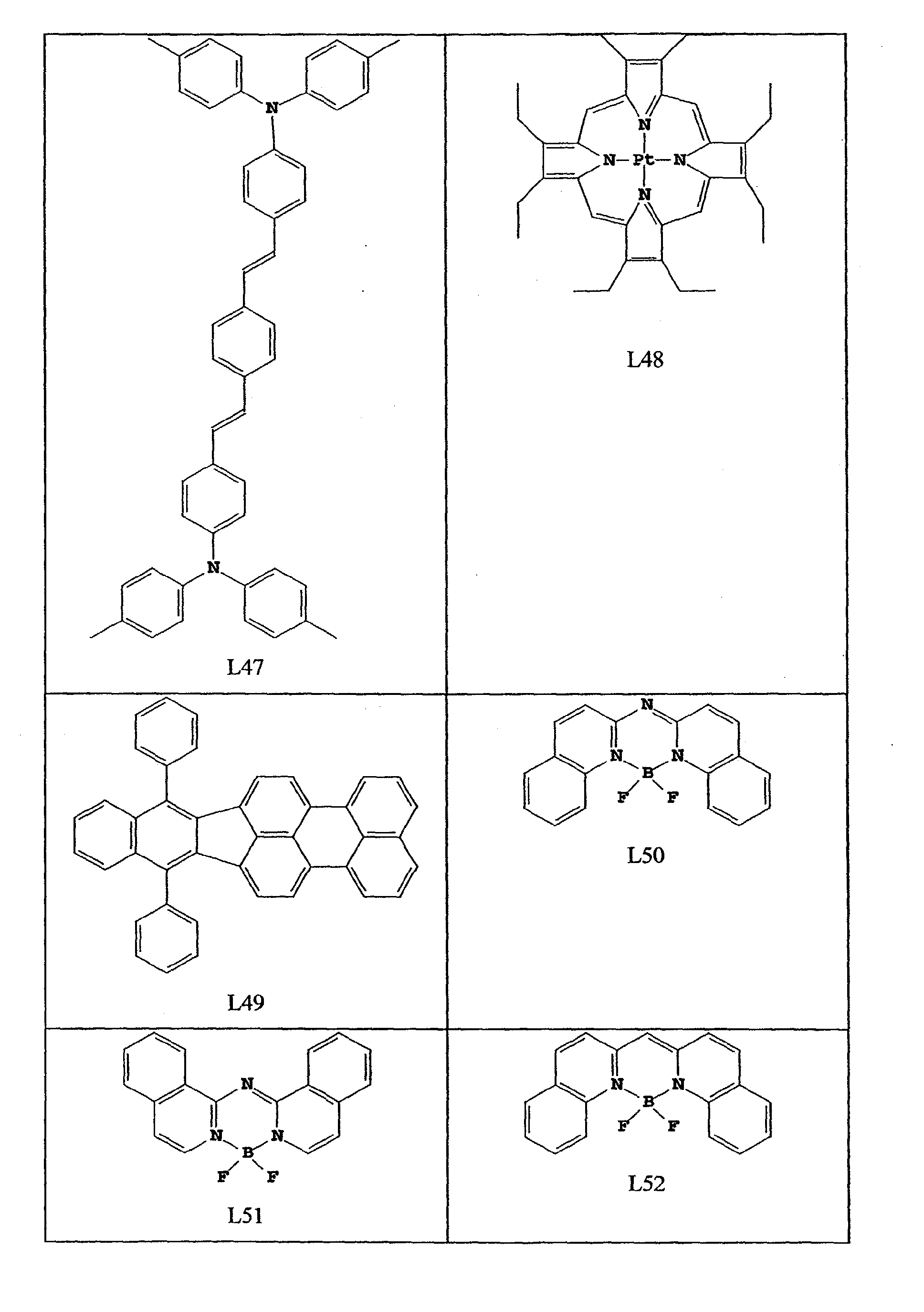

The LEL may further comprise stabilizing compounds such as naphthopyrenes and indenoperylenes.

### Electron-Transporting Layer (ETL)

Preferred thin film-forming materials for use in forming the electron-transporting layer **111** of the organic EL devices of this invention are metal chelated oxinoid compounds, including chelates of oxine itself (also commonly referred to as 8-quinolinol or 8-hydroxyquinoline). Such compounds help to inject and transport electrons and exhibit both high levels of performance and are readily fabricated in the form of thin films. Exemplary of contemplated oxinoid compounds are those satisfying structural formula (E), previously described.

Other electron-transporting materials include various butadiene derivatives as disclosed in US 4,356,429 and various heterocyclic optical brighteners as described in US 4,539,507. Benzazoles satisfying structural formula (G) are also useful electron transporting materials. Triazines are also known to be useful as electron transporting materials.

### Cathode

When light emission is viewed solely through the anode, the cathode **113** used in this invention can be comprised of nearly any conductive material. Desirable materials have good film-forming properties to ensure good contact with the underlying organic layer, promote electron injection at low voltage, and have good stability. Useful cathode materials often contain a low work function metal (< 4.0 eV) or metal alloy. One preferred cathode material is comprised of a Mg:Ag alloy wherein the percentage of silver is in the range of 1 to 20 %, as described in U.S. Patent No. 4,885,221. Another suitable class of cathode materials includes bilayers comprising a thin electron-injection layer (EIL) in contact with the organic layer (e.g., ETL) which is capped with a thicker layer of a conductive metal. Here, the EIL preferably includes a low work function metal or metal salt, and if so, the thicker capping layer does not need to have a low work function. One such cathode is comprised of a thin layer of LiF followed by a thicker layer of Al as described in U.S. Patent No. 5,677,572. Other useful cathode material sets include, but are not limited to, those disclosed in U.S. Patent Nos. 5,059,861; 5,059,862, and 6,140,763.

When light emission is viewed through the cathode, the cathode must be transparent or nearly transparent. For such applications, metals must be thin or one must use transparent conductive oxides, or a combination of these materials. Optically transparent cathodes have been described in more detail in US 4,885,211, US 5,247,190, JP 3,234,963, US 5,703,436, US 5,608,287, US 5,837,391, US 5,677,572, US 5,776,622, US 5,776,623, US 5,714,838, US 5,969,474, US 5,739,545, US 5,981,306, US 6,137,223, US 6,140,763, US 6,172,459, EP 1 076 368, US 6,278,236, and US 6,284,3936. Cathode materials are typically deposited by evaporation, sputtering, or chemical vapor deposition. When needed, patterning can be achieved through many well known methods including, but not limited to, through-mask deposition, integral shadow masking as described in US 5,276,380 and EP 0 732 868, laser ablation, and selective chemical vapor deposition.

### Other Useful Organic Layers and Device Architecture

In some instances, layers **109** and **111** can optionally be collapsed into a single layer that serves the function of supporting both light emission and electron transportation. It also known in the art that emitting dopants may be added to the hole-transporting layer, which may serve as a host. Multiple dopants may be added to one or more layers in order to create a white-emitting OLED, for example, by combining blue- and yellow-emitting materials, cyan- and red-emitting materials, or red-, green-, and blue-emitting materials. White-emitting devices are described, for example, in EP 1 187 235, US 20020025419, EP 1 182 244, US 5,683,823, US 5,503,910, US 5,405,709, and US 5,283,182.

Additional layers such as electron or hole-blocking layers as taught in the art may be employed in devices of this invention. Hole-blocking layers are commonly used to improve efficiency of phosphorescent emitter devices, for example, as in US 20020015859.

This invention may be used in so-called stacked device architecture, for example, as taught in US 5,703,436 and US 6,337,492.

### Deposition of organic layers

The organic materials mentioned above are suitably deposited through sublimation, but can be deposited from a solvent with an optional binder to improve film formation. If the material is a polymer, solvent deposition is usually preferred. The material to be deposited by sublimation can be vaporized from a sublimator "boat" often comprised of a tantalum material, e.g., as described in US 6,237,529, or can be first coated onto a donor sheet and then sublimed in closer proximity to the substrate. Layers with a mixture of materials can utilize separate sublimator boats or the materials can be pre-mixed and coated from a single boat or donor sheet. Patterned deposition can be achieved using shadow masks, integral shadow masks (US 5,294,870), spatially-defined thermal dye transfer from a donor sheet (US 5,688,551, US 5,851,709 and US 6,066,357) and inkjet method (US 6,066,357).

### Encapsulation

Most OLED devices are sensitive to moisture or oxygen, or both, so they are commonly sealed in an inert atmosphere such as nitrogen or argon, along with a desiccant such as alumina, bauxite, calcium sulfate, clays, silica gel, zeolites, alkaline metal oxides, alkaline earth metal oxides, sulfates, or metal halides and perchlorates. Methods for encapsulation and desiccation include, but are not limited to, those described in U.S. Patent No. 6,226,890. In addition, barrier layers such as SiOx, Teflon, and alternating inorganic/polymeric layers are known in the art for encapsulation.

### Optical Optimization

OLED devices of this invention can employ various well-known optical effects in order to enhance its properties if desired. This includes optimizing layer thicknesses to yield maximum light transmission, providing dielectric mirror structures, replacing reflective electrodes with light-absorbing electrodes, providing anti glare or anti-reflection coatings over the display, providing a polarizing medium over the display, or providing colored, neutral density, or color conversion filters over the display. Filters, polarizers, and anti-glare or anti-reflection coatings may be specifically provided over the cover or as part of the cover.

The entire contents of the patents and other publications referred to in this specification are incorporated herein by reference.

### EXAMPLES

The invention and its advantages are further illustrated by the specific examples which follow.

### Example1-Preparation of Inv-1

a) Preparation of 1,4-cyclohexadiene-1,4-dicarboxylic acid, 2,5 bis(phenylamino)-, dimethyl ester: A 50 g (215 mmol, 1eq) sample of 1,4-cyclohexanedione-2,5-dicarboxylate was combined with a slight excess of aniline (45mL) in a 250mL round bottom flask. The resulting neat mixture was brought to 80-90 °C for 4 h via heating mantle. Usually the product precipitates out within the 4 hours of heating. The mixture is then removed from the heat, and while warm, methanol is added, and the solid slurried in methanol. The product is isolated by filtration, washed with 100mL methanol, then 50mL of P950 ligroin, for drying to yield 77g (95%) of clean material. The product can be used for the next step, without purification.
b) Preparation of 1,4-benzenedicarboxylic acid, 2,5-bis(phenylamino)-, dimethyl ester: A 50 g sample of the above intermediate was partly dissolved in 1L of toluene, in a 2L, 3 neck round bottom flask. A reflux condenser was attached to one joint, one joint was plugged and the other was connected to a flow of air. The vigorously stirred mixture was brought just below reflux by means of a heating mantle, and a flow of air was generated at the surface of the liquid. After 4 h TLC showed no byproducts, and a 50% clean conversion of the cyclohexene intermediate to the aromatic product. The reaction was complete after 4 additional hours, with very little impurities present. The mixture was concentrated and the red solid residue was suspended in 50 mL of MeOH, the solid was filtered off and washed with another portion of MeOH (50 mL), then P950 ligroin, to yield 90% (44.8 g) of a bright orange product. More product can be recovered if the mother liquor is concentrated, chilled and the process above repeated.
c) Preparation of 1,4-benzenedicarboxylic acid, 2,5-bis (N,N'-diphenylamino), -dimethyl ester: A 40 g (97 mmol, 1eq) sample of 1,4-benzenedicarboxylic acid, 2,5-bis(phenylamino)-, dimethyl ester, 65 mL (large excess necessary for ease of stirring) of iodobenzene, 27 g (194 mmol, 2 eq) of potassium carbonate, 12.3 g (197 mmol, 2 eq) of copper, and 3 g of copper(I)iodide were combined in a 250 mL round bottom flask. The resulting mixture was too thick to stir efficiently, so about 10 mL of toluene were also added; the toluene gradually evaporated off. The mixture was refluxed overnight (around 150-160 °C); the originally red mixture turned greenish-brown. TLC indicated one spot with very little baseline impurities. The thick slurry was cooled to room temperature, dissolved in methylene chloride and the inorganic solids were removed by filtration. The solid residue was repeatedly washed with methylene chloride, and the washes were concentrated to a syrup. The concentrate was chilled in ice, the resulting solid was isolated by filtration, washed with MeOH, then with P950 ligroin. The bright yellow product was obtained in 85%yield (47g).
d) Preparation of quino (2,3-b)acridine-7,14-dione, 5,12-dihydro-5,12-diphenyl or N, N-diphenyl quinacridone: A 167 g sample of the precursor above was suspended in about 200mL of methane sulfonic acid. The thick suspension was quickly brought to 140 °C and the resulting blue mixture was stirred at the temperature for 4h. The thick reaction mixture was cooled and slowly poured over ice (in a 1L beaker), with vigorous stirring. The resulting reddish-brown suspension was left to stand such that the solid would settle and the aqueous phase could be decanted. The process was repeated twice, then one more time using H₂O and Na₂CO₃ (aq, sat), in a 1:1 ratio. The solid was then isolated by filtration to yield 95% of red-brown crude product.

### Example 2 - Inventive EL devices

An EL device satisfying the requirements of the invention was constructed in the following manner:

A glass substrate coated with a 42 nm layer of indium-tin oxide (ITO) as the anode was sequentially ultrasonicated in a commercial detergent, rinsed in deionized water, degreased in toluene vapor and exposed to oxygen plasma for about 1 min.
a) Over the ITO was deposited a 1 nm fluorocarbon hole-injecting layer (CFx) by plasma-assisted deposition of CHF₃.
b) A hole-transporting layer of *N,N'*-di-1-naphthalenyl-*N,N'*-diphenyl-4, 4'-diaminobiphenyl (NPB) having a thickness of 75 nm was then evaporated from a tantalum boat.
c) A 37.5nm light-emitting layer of Alq doped with 0.5 % Inv-1 was then deposited onto the hole-transporting layer. These materials were coevaporated from tantalum boats. Herein, doping percentage is reported based on volume/volume ratio..
d) A 30 nm electron-transporting layer of tris(8-quinolinolato)aluminum (III) (Alq) was then deposited onto the light-emitting layer. This material was also evaporated from a tantalum boat.
e) On top of the Alq layer was deposited a 220 nm cathode formed of a 10:1 volume ratio of Mg and Ag.

The above sequence completed the deposition of the EL device. The device was then hermetically packaged in a dry glove box for protection against ambient environment.

### Examples 3 - 13. Comparative EL devices

EL devices of Examples 3 - 12 were fabricated in the same manner as Example 2 except that, in place of Inv-1, other quinacridone derivatives not part of this invention, were used as dopants. The dopant % are reported in Table 1.

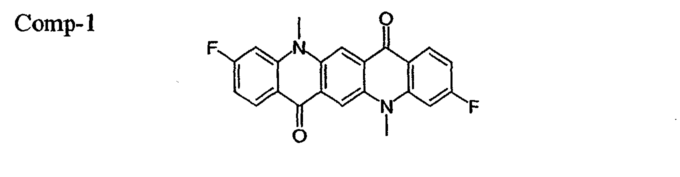

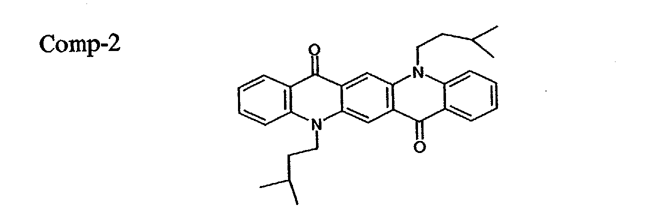

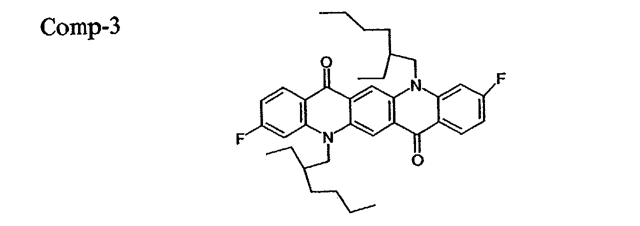

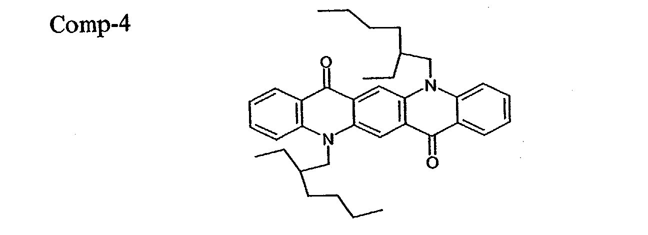

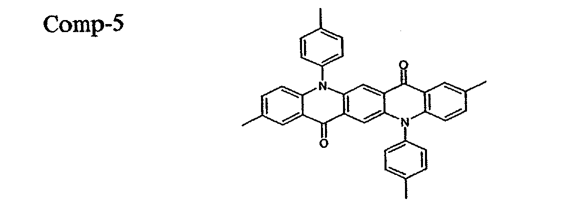

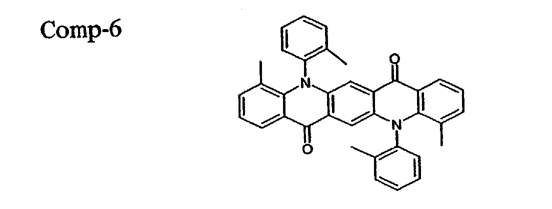

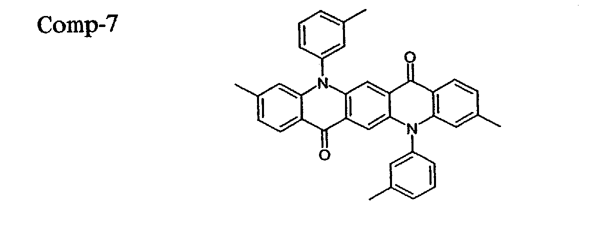

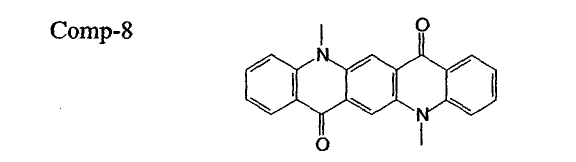

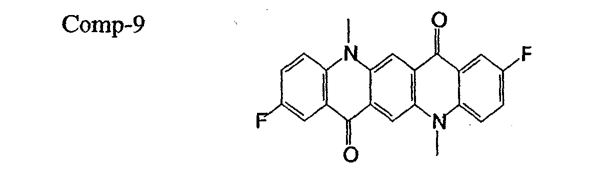

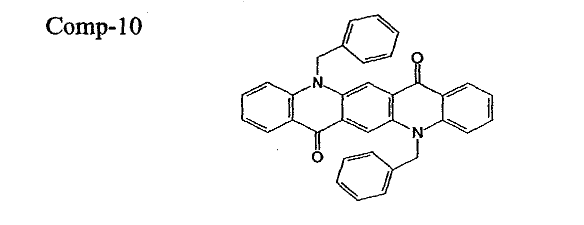

The cells thus formed in Examples 2 - 12 were tested for efficiency in the form of luminance yield (cd/A) measured at 20 mA/cm². CIE color x and y coordinates were determined. It is desirable to have a luminance yield of at least about 7 cd/A and preferably greater than about 8 cd/A. An acceptable green for a high quality full color display device has CIEx of no more than about 0.35 and CIEy no less than about 0.62. The luminance loss was measured by subjecting the cells to a constant current density of 20mA/cm² at 70 °C, to various amounts of time that are specified for each individual cell/example. Useful stability for use in a display device is desirably less than about 40% loss after about 300 hours of these accelerated aging conditions. All of these testing data are shown in Table 1

**Table 1.**

| Type | Structure | % dopant | cd/A | CIEx | CIEy | Luminance Loss (hours, % loss) |
|---|---|---|---|---|---|---|
| Example 2 Inventive | Inv-1 | 0.5 | 8.5 | 0.327 | 0.639 | 300h 23 % |
| Example 3 Comparative | Comp-1 | 0.5 | 6.5 | 0.313 | 0.638 | 220h 30 % |
| Example 4 comparative | Comp-2 | 2 | 9.8 | 0.4 | 0.586 | 325h 40 % |
| Example 5 comparative | Comp-3 | 1 | 10.2 | 0.434 | 0.555 | 325h 34% |
| Example 6 comparative | Comp-4 | 2 | 8.5 | 0.394 | 0.592 | 270h 42 % |
| Example 7 comparative | Comp-5 | 0.8 | 8.75 | 0.368 | 0.609 | 220h 33 % |
| Example 8 comparative | Comp-6 | 0.6 | 7.27 | 0.314 | 0.644 | 270h 43 % |
| Example 9 comparative | Comp-7 | 0.8 | 7.26 | 0.33 | 0.632 | 200h 50 % |
| Example 10 comparative | Comp-8 | 0.6 | 8.36 | 0.370 | 0.604 | 220h 30 % |
| Example 11 Comparative | Comp-9 | 0.4 | 6.13 | 0.423 | 0.553 | 220h 28 % |
| Example 12 comparative | Comp-10 | 0.6 | 9.32 | 0.336 | 0.633 | 200h 50 % |

From the summary above it is evident that any structure with a methyl substituent on the nitrogen or aromatic rings does not provide an optimum combination of color, stability and efficiency. The same is true for the N-alkylated analogs of quinacridones. In addition to the high luminance yields demonstrated by Inv-1 (N,N'-diphenylquinacridone), the stability of this compound is superior to all comparative examples.

### Example 13 - inventive.

An EL device was constructed as described in Example 1 except that the light-emitting layer utilized TBADN as host. This device had an initial luminance efficiency of 6.8 cd/A measured at 20 mA/cm². A luminance loss of 23% was measured when subjecting the cell to a constant current density of 20mA/cm² at 70 °C for 280 hours.

### Example 14 - comparative

An EL device was constructed as in Example 13 except that Comp-1 was used as the dopant. This device had an initial luminance of 4.9 cd/A measured at 20 mA/cm². A luminance loss of 43% was measured when subjecting the cell to a constant current density of 20mA/cm² at 70 °C for 280 hours.

Examples 13 and 14 demonstrate that the superior performance of the inventive compound as dopant is realized using a host other than Alq.

### Examples 15 - 19:

A series of EL devices was constructed as described in Example 2, except that in Step c, a level series of TBADN was used along with Alq as the host matrix. The % TBADN values are reported in Table 2, and the balance is Alq. The cells thus formed in Examples 15 - 19 were tested for efficiency in the form of luminance yield (cd/A) measured at 20 mA/cm². CIE color x and y coordinates were determined. The luminance loss was measured by subjecting the cells to a constant current density of 20mA/cm² at 70 °C for 290 hours, or at room temperature for 340 hours. All of these testing data are shown in Table 2.

**Table 2.**

| Type | % Inv-1 | cd/A | CIEx,y | 70 °C luminance loss (%) | % TBADN | Room temp luminance loss (%) |
|---|---|---|---|---|---|---|
| Example 15 (comparative) | 0 | 2.72 | .335, .552 | 40 | 0 | 12 |
| Example 16 (inventive) | 0.5 | 9.71 | .309, .652 | 32 | 0 | 16 |
| Example 17 (inventive) | 0.5 | 6.56 | .305, .648 | 22 | 25 | 6 |
| Example 18 (inventive) | 0.5 | 7.6 | .306, .648 | 19 | 50 | 6 |
| Example 19 (inventive) | 0.5 | 8.05 | .304, .648 | 18 | 75 | 6 |

Example 16 was brighter and less stable than usual, but the data show that addition of TBADN improves the stability. Interestingly, low levels of TBADN yield a fairly significant drop in luminance, but increasing levels show the luminance to largely recover with a concurrent increase in stability. A desirable TBADN percentage is greater than 50 % but less than 100 %. Preferably, this range is 70 - 90%.

The device of the invention includes those wherein the dopant is present in an amount of less than a 2 wt% ratio to host and includes those of the formula of the invention R₁ and R₂ are hydrogen or independently selected from halogen, aryl, an aromatic heterocycle, or a fused aromatic or heteroaromatic ring; wherein R₃ through R₆ represents hydrogen or one or more substituents independently selected from halogen, aryl, and an aromatic heterocycle; wherein R₁ - R₆ are independently selected hydrogen, phenyl, biphenyl, or naphthyl groups.

Also included are the device wherein the dopant is present in an amount of 0.1 to 1 wt % ratio to host, and where the device includes an electron transporting layer and a hole transporting layer, and includes a display device comprising the OLED device of claim 1.

## Claims

1. An OLED device comprising a non-gallium host compound and a green light emitting dopant wherein the dopant comprises an N,N'-diarylquinacridone compound optionally containing on the two aryl groups and the quinacridone nucleus only substituent groups having Hammett's σ constant values at least 0.05 more positive than that for a corresponding methyl group, such substituent groups including up to two substituent groups directly on the carbon members of the quinacridone nucleus, provided that said substituent groups do not form a ring fused to the five-ring quinacridone nucleus.

2. The device of claim 1 wherein the N,N'-diarylquinacridone compound is unsubstituted.

3. The device of claim 1 or 2 wherein the diaryl groups are diphenyl groups.

4. The device of any one of claims 1-3 wherein the host comprises an aluminum complex, an anthracene compound, or a distyrylarylene derivative.

5. The device of any one of claims 1-3 wherein the host comprises Alq, ADN, or TBADN.

6. The device of any one of claims 1-3 wherein the host comprises a co-host comprising Alq and TBADN.

7. The device of any one of claims 1-6 wherein the dopant is present in an amount of less than a 10 wt%, ratio to host.

8. The device of claim 7 wherein the dopant is present in an amount of 0.1 to 1wt% ratio to host.

9. The device of claim 1 wherein the substituents are selected so that the device emits green light having a CIEx value less than 0.35, a CIEy value greater than 0.62, and a luminance efficiency greater than 7 cd/A when applied with a current density of 20 mA/cm².

10. The OLED device of claim 1 wherein the dopant has the following formula: 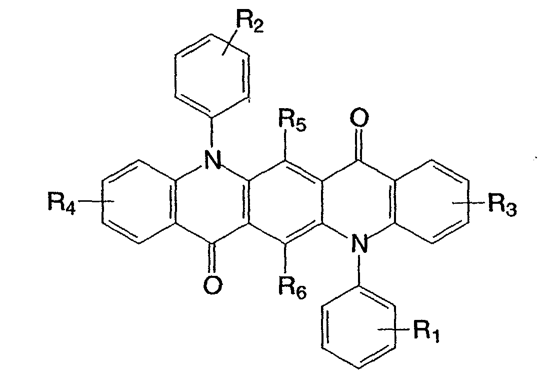 wherein, R₁ and R₂ represent one or more independently selected hydrogen or substituent groups having Hammett's σ constant values at least 0.05 more positive than that for a corresponding methyl group and each of R₃ through R₆ represents hydrogen or up to two substituents as selected for R₁ above.

11. An OLED device comprising a cathode, an anode and having located between the cathode and electrode a non-gallium host compound and a green light emitting dopant as described in claim 1.
